Europäisches Patentamt

European Patent Office

Office européen des brevets

(11) Veröffentlichungsnummer: **0 451 317 A1**

## (12) EUROPÄISCHE PATENTANMELDUNG

(21) Anmeldenummer: 90108009.3

(22) Anmeldetag: 27.04.90

(51) Int. Cl.⁵: **A61L 11/00**

(30) Priorität: 13.04.90 DE 4012057

(43) Veröffentlichungstag der Anmeldung:
16.10.91 Patentblatt 91/42

(84) Benannte Vertragsstaaten:
AT BE CH DE DK ES FR GB GR IT LI LU NL SE

(71) Anmelder: **VETCO SANITEC GMBH**
**Elwerathstrasse 2**
**W-3101 Nienhagen(DE)**

(72) Erfinder: **Mertsch, Joachim, Dipl.-Ing.**
**Drosselweg 14**
**W-3101 Nienhagen(DE)**

(74) Vertreter: **Sparing Röhl Henseler**
**Patentanwälte European Patent Attorneys**
**Rethelstrasse 123**
**W-4000 Düsseldorf 1(DE)**

(54) **Verfahren und Vorrichtung zum Desinfizieren oder Sterilisieren von infektiösem Müll.**

(57) Die Erfindung betrifft ein Verfahren und eine Vorrichtung zum Desinfizieren oder Sterilisieren von infektiösem Müll, wobei der Müll zunächst zerkleinert, befeuchtet und durch Bestrahlen mit Mikrowellen thermisch behandelt wird. Zur sicheren und schnellen Desinfektion oder Sterilisation in schneller und wirtschaftlicher Weise ist vorgesehen, daß der zerkleinerte Müll chargenweise in eine verschließbare Mikrowellenkammer eingefüllt und dabei durch Beaufschlagung mit Wasserdampf befeuchtet und vorgeheizt wird. Danach wird durch zumindest zeitweilig gleichzeitiges Beaufschlagen mit Wasserdampf und Bestrahlen mit Mikrowellen der Müll auf eine Wärembehandlungstemperatur von über 120° C bei mindestens 2 bar (absolut) aufgeheizt und dort während einer Mindestdauer bis zur Desinfektion oder Sterilisation des Mülls gehalten.

### FIG. 1

Die Erfindung betrifft ein Verfahren zum Desinfizieren oder Sterilisieren von infektiösem Müll nach dem Oberbegriff des Anspruchs 1 und eine Vorrichtung dazu.

Für ein derartiges Verfahren zur Behandlung von infektiösem Müll mit Hilfe von Mikrowellen ist aus der DE-PS 35 05 570 eine Vorrichtung bekannt, die als kompakte Entsorgungsanlage einen Eingabeabschnitt mit einem Müllzerkleinerer und eine Mikrowellenkammer zur Wärmebehandlung umfaßt. In der Mikrowellenkammer ist ein Transportweg vorgesehen, der von einem mikrowellendurchlässigen Gehäuse umgeben ist. Entlang des Transportweges sind mehrere Mikrowellensender angeordnet, durch die der zerkleinerte und befeuchtete Infektionsmüll bis zur Sterilisation mit Mikrowellen bestrahlt wird. Gegen das Entweichen von Keimen und Dampf ist die gesamte Vorrichtung nach außen abgeschlossen.

Bei diesem mit der bekannten Vorrichtung durchgeführten Verfahren hat sich gezeigt, daß die für eine Desinfektion und insbesondere Sterilisation erforderliche Abtötung der Mikroorganismen sowie die Inaktivierung der Viren lange Durchlaufzeiten der Wärmebehandlung erfordert. Der Energieaufwand für eine derart durchgeführte Wärmebehandlung ist folglich hoch.

Aufgabe der Erfindung ist es daher, ein Verfahren und eine Vorrichtung der genannten Art zu schaffen, die eine sichere Desinfektion oder Sterilisation in schneller und wirtschaftlicher Weise erlauben.

Diese Aufgabe wird gemäß dem kennzeichnenden Teil des Anspruchs 1 bzw 3 gelöst.

Hierdurch werden ein Verfahren und eine Vorrichtung zur Durchführung des Verfahrens geschaffen, die durch gleichzeitige Anwendung von feuchter Hitze, Überdruck und Mikrowellen die Wärmebehandlung zur Desinfektion oder Sterilisation wesentlich verkürzen und dabei auch das Überleben resistenter Mikroorganismen und Viren verhindern. Das Einspeisen von Wasserdampf während des Einfüllvorganges stellt bereits eine schnelle und gute Vorheizung unter Einbringung von Feuchtigkeit sicher. Die anschließende Aufheiz- und Haltephase ermöglicht in dem feuchten Überdruckmedium einen wirksamen Angriff der Mikrowellen. Eine wesentliche Verminderung bzw. Abtötung aller Mikroorganismen usw. innerhalb weniger Minuten ist so möglich.

Vorzugsweise werden die Temperaturen in der Mikrowellenkammer zwischen 130° und 145° C eingestellt. Zur möglichst gleichmäßigen Einspeisung des Wasserdampfs können mehrere Injektionsstellen mit Abstand entlang der Mikrowellenkammer verteilt sein. Eine Verteilung der Mikrowellen durch Reflexion kann durch eine metallische Transportschnecke erreicht werden. Für einen gleichmäßigen Chargendurchfluß kann die Mikrowellenkammer eingangsseitig mit einer Transferkammer und ausgangsseitig mit einer Ausförderkammer ausgestattet sein, die jeweils gleiche Chargen wie die Mikrowellenkammer aufnehmen können.

Weitere Ausgestaltungen der Erfindung sind der nachfolgenden Beschreibung und den Unteransprüchen zu entnehmen.

Die Erfindung wird nachstehend anhand des in den beigefügten Abbildungen dargestellten Ausführungsbeispiels näher erläutert.

Fig. 1     zeigt einen Längsschnitt einer Vorrichtung zum Desinfizieren oder Sterilisieren von infektiösem Müll,

Fig. 2     zeigt einen Querschnitt der Vorrichtung gemäß Fig. 1.

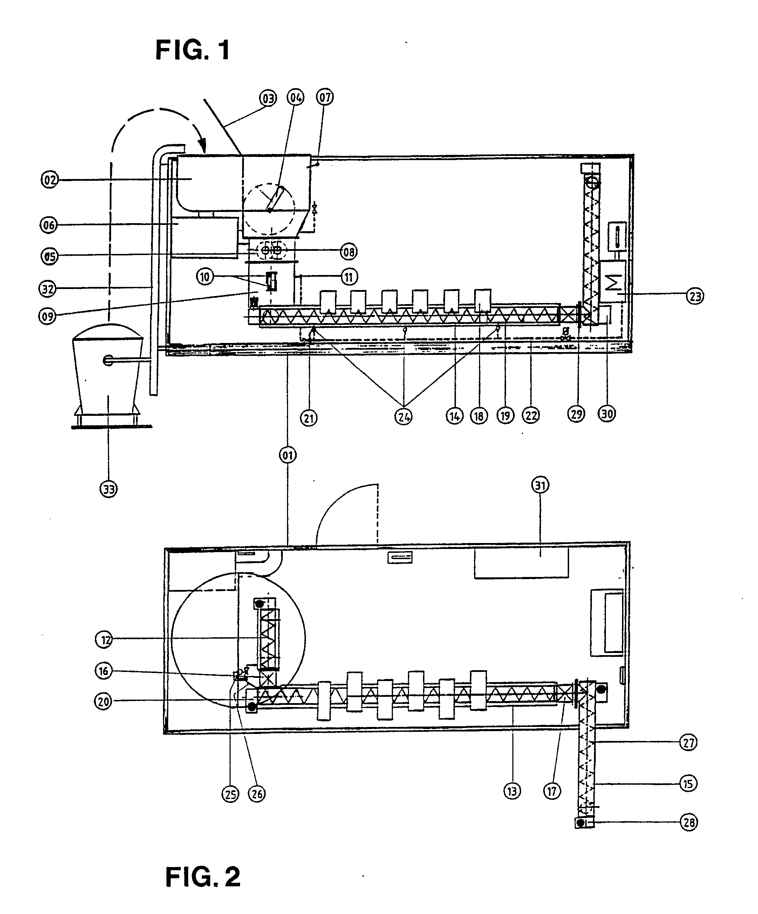

Figur 1 und Figur 2 zeigen eine Vorrichtung zum Behandeln, insbesondere Sterilisieren, von infektiösem Müll, die in einen Container 1 eingebaut ist.

Ein Eingabeabschnitt umfaßt zur Aufnahme von infektiösem Müll eine Einfüllschleuse 2, die über einen Deckel 3 fluiddicht verschließbar ist. In der Einfüllschleuse 2 ist eine Drehschwinge 4 angeordnet, die den Müll vorzerkleinert und einem bodenseitig anschließenden Müllzerkleinerer 5 zuführt. Mittels einer Absauganlage 6 kann das Austreten von Keimen oder Dampf verhindert werden. Desweiteren ist mindestens eine Injektionsstelle 7 für das Einspeisen von Wasserdampf in die Einfüllschleuse 2 eingelassen. Die Schleusenwände sind vorzugsweise wärmeisoliert, können aber auch indirekt beheizbar ausgebildet sein.

Der Müllzerkleinerer 5 besteht aus einem Schneidwerk mit zwei gegenläufigen Messerwellen 8 und zerschnitzelt das durch den Einfülltrichter 2 mit Hilfe der Drehschwinge 4 zugeführte Abfallmaterial.

An den Müllzerkleinerer 5 schließt sich ein Transferbehälter 9 an, der als Zwischenspeicher für den zerkleinerten Müll genutzt wird. An den Seiten des Transferbehälters 9 befinden sich Füllstandswächter 10 für einen minimalen oder maximalen Füllstand. Der Zerkleinerer 5 wird so getaktet, daß der Füllstand im Transferbehälter 9 immer zwischen Minimum und Maximum pendelt. Zusätzlich ist ein Flüssigkeits-Füllstandswächter 11 installiert, der bei zu hohem Flüssigkeitsstand Alarm gibt. Aus dem Boden des Transferbehälters 9 transportiert eine Transferschnecke 12 (vgl. Fig. 2) den zerkleinerten Müll zu einer Mikrowellenkammer 13, die eingangsseitig mit dem Transferbehälter 9 verbunden ist. Die Transportschnecke 12 ist mit einem Schneckenbaum ausgerüstet und wird durch einen Getriebemotor angetrieben, der durch die Füllstandswächter 10 und die Mikrowellenkammer 13 gesteuert wird.

Überführt wird der zerkleinerte Müll in die Mikrowellenkammer 13 über die Transportschnecke 12 gemäß einem festgelegten Arbeitszyklus.

Die Mikrowellenkammer 13 besteht aus einer rohr- bzw. kanalförmigen Druckkammer 14, die zwischen dem Transferbehälter 9 und einer Ausförderkammer 15 angeordnet ist. Dabei ist der eingangs- und ausgangsseitige Anschluß der Mikrowellenkammer 13 mittels jeweils einer Absperrarmatur 16, 17 fluiddicht verschließbar. Längs der Druckkammer 14 sind mehrere Mikrowellensender 18 mit Abstand zueinander angeordnet. Die Druckkammer 14 ist metallische ausgebildet oder mit einem für Mikrowellen undurchlässigen Material ummantelte und weist für die Einkoppelung der Mikrowellenstrahlen einzelne Ausschnitte bzw. Fenster auf, die mit mikrowellendurchlässigem Material abgedichtet sind. An die Ausschnitte bzw. Fenster sind die Mikrowellensender 18 über ein Hohlleiter- und Resonanzkammersystem fluiddicht und druckbelastbar angeschlossen. Vorzugsweise strahlt jeder Mikrowellensender 18 über zwei zueinander in Umfangsrichtung verdrehte Ausschnitte Mikrowellen in die Mikrowellenkammer 13 ein. Die Arbeitsfrequenz der Mikrowellensender 18 kann geeignet gewählt werden und beträgt hier 2450 MHz. In der Druckkammer 14 ist eine Fördereinrichtung 19, hier eine Transportschnecke (Sterilisationsschnecke), angeordnet, die sich vorzugsweise über die gesamte Länge der Druckkammer 14 erstreckt. Diese Sterilisationsschnecke 19 ist mit einem Schneckenbaum ausgerüstet und besteht aus einem die Mikrowellen reflektierenden Material. Durch einen Getriebemotor 20 ist sie angetreibbar, und zwar zum Befüllen der Mikrowellkammer vorwärtsrotierend und bei isolierter Mikrowellenkammer (geschlossene Absperrarmaturen 16, 17) vorwärts- und rückwärtsrotierend.

Entlang der Druckkammer 14 sind weiterhin mehrere mit Abstand zueinander angeordnete Düsen 21 in die Wandung des Durchgangsgehäuses 14 eingelassen. Diese Düsen 21 sind Injektionsstellen für das Einspeisen von Wasserdampf und sind hierzu über ein Speiseleitungssystem 22 an einen Dampferzeuger 23 angeschlossen. Zumindest eine Düse 21 befindet sich im Eingangsbereich der Mikrowellenkammer 13 und vorzugsweise ist jede Düse 21 über ein eigenes Ventil 24 zu- und abschaltbar. Mittels eines Druckhalteventils 25 in einer Abluftleitung 26 der Mikrowellenkammer 13 kann der durch Einspeisung von Wasserdampf in der Mikrowellenkammer 13 einstellbare Druck reguliert und konstant gehalten werden.

Die Abmessungen der Mikrowellenkammer 13 sind so gewählt, daß Chargen von infektiösem Müll zwischen vorzugsweise 200 und 400 l aufgenommen werden können.

Die ausgangsseitig an die Mikrowellenkammer 13 angeschlossene Ausförderkammer 15 besteht aus einem rohrförmigen Durchgangsgehäuse 27, in dem eine Transportschnecke 28 (Entleerungsschnecke) angeordnet ist und über einen Getriebemotor 28 angetrieben wird. Gehäuse 27 und Schnecke 28 bestehen vorzugsweise aus Edelstahl. Für einen besseren mobilen Betrieb der Vorrichtung ist die Ausförderkammer 15 über ein Drehgelenk 29 mit der Mikrowellenkammer 13 verbunden. Folglich kann die Ausförderkammer 15 mit einem Getriebemotor 30 bei Nicht-Betrieb hochgeschwenkt werden (Fig. 1) und bei Betrieb aus dem Container 1 herausgeschwenkt werden (Fig.2). Die Abmessungen der Ausförderkammer 15 werden so gewählt, daß eine zuvor in der Mikrowellenkammer 13 behandelte Charge vollständig aufgenommen werden kann.

Für die elektrische Steuerung ist eine speicherprogrammierbare Steuerung vorgesehen. Sie befindet sich in einem Schaltschrank 31 und enthält ein Programm für Automatik- und Handbetrieb, d.h. für Schalter- und Sensorabfragen, Motoren- und Mikrowellensender-Steuerung, Überwachung und Ansteuerung von Anzeigeeinrichtungen.

Die Funktionsweise der vorstehend beschriebenen Vorrichtung und das damit durchführbare Verfahren zur Behandlung von infektiösem Müll wird nachfolgend beschrieben.

Zum Befüllen der Vorrichtung wird zunächst die eingangsseitige Absperrarmatur 16 der Mikrowellenkammer 13 geöffnet und die ausgangsseitige Absperrarmatur 17 vorzugsweise geschlossen. Die Dampfdüsen 21 sind abgeschaltet. Der infektiöse Müll, der beispielsweise in einem Sammelbehälter 33 gelagert ist, wird in den Einfülltrichter 4 geworfen und der Deckel 3 geschlossen. Für das Anheben des Sammelbehälters 33 kann eine zusätzliche Hub- und Kippeinrichtung 32 vorgesehen sein. Durch Betätigung der Drehschwinge 4 wird der eingefüllte Müll dem Müllzerkleinerer 5 unter einer Vorzerkleinerung zugeführt. Der zerkleinerte Müll fällt dann in den Transferbehälter 9. Die Füllstandsüberwachung regelt den Zerkleinerungsvorgang, indem bei Erreichen des oberen Füllstandswächters 10 abgeschaltet wird. Die einer gewählten Charge entsprechende Menge an zerkleinertem Müll wird mittels der Transportschnecke 12 aus dem Transferbehälter 9 heraustransportiert und über die vorwärtsrotierende Sterilisationsschnecke 19 in die Mikrowellenkammer 13 eingefüllt. Mit Beginn des Einfüllens des Mülls in die Mikrowellenkammer 13 wird mit der Einspeisung von Wasserdampf begonnen. Das Injizieren des Wasserdampfes führt zu einem Befeuchten und Vorheizen des Mülls auf etwa 50 bis 60° C während eines Einfüllvorganges von etwa 3 bis 10 Minuten für Chargen zwischen 200 und 400 l.

Ist die Mikrowellenkammer 13 mit zerkleinertem Müll gefüllt, wird die eingangsseitige Absperrarmatur 16 und gegebenenfalls die ausgangsseitige geschlossen. Die Mikrowellenkammer 13 ist damit isoliert vom Transferbehälter 9 und der Ausförderkammer 15. Die weitere Dampfinjektion mit vorzugsweise gesättigtem Dampf führt zum Aufbau eines Drucks, der über das Druckhalteventil 25 konstant gehalten wird. Der gesättigte Dampf des Dampferzeugers 23 hat einen Arbeitsdruck bis zu 6 bar, d.h. eine Arbeitstemperatur von 165° C.

Zusätzlich werden Mikrowellenstrahlen in die Mikrowellenkammer 13 eingekoppelt. Während der Mikrowellenbehandlung rotiert die Sterilisationsschnecke 19 vor und zurück, um Reflexionsrückstrahlung auf die Mikrowellensender 18 zu verhindern. Ist der gewählte Druck von mindestens 2 bar (absolut) in der Mikrowellenkammer 13 erreicht, erfolgt die Abschaltung der Dampfbeaufschlagung. Mittels Mikrowellenbestrahlung kann die gewünschte Behandlungstemperatur über 120° C, vorzugsweise 130° bis 145° C, geregelt werden. Diese Temperatur wird für eine bestimmte Mindestdauer gehalten werden, um die Abtötung der Mikroorganismen usw. sicherzustellen. Danach wird die Absperrarmatur 17 geöffnet, und die Charge behandelter Müll in die Ausförderkammer 15 entleert. Die ausgangsseitige Absperrarmatur 17 der Mikrowellenkammer 13 wird wieder geschlossen, um eine neue Kontaminierung des bereits behandelten Mülls zu unterbinden. In der Ausführkammer 15 kann der Müll sich noch abkühlen und wird bei Übergabe der nachfolgenden Charge aus der Mikrowellenkammer 13 als desinfizierter bzw. sterilisierter Müll für einen Abtransport ausgeworfen.

## Patentansprüche

1. Verfahren zum Desinfizieren oder Sterilisieren von infektiösem Müll, bei dem der Müll zerkleinert, befeuchtet und durch Bestrahlen mit Mikrowellen thermisch behandelt wird, dadurch gekennzeichnet, daß der zerkleinerte Müll chargenweise in eine verschließbare Mikrowellenkammer eingefüllt und dabei durch Beaufschlagen mit Wasserdampf befeuchtet und vorgeheizt wird und danach durch zumindest zeitweilig gleichzeitiges Beaufschlagen mit Wasserdampf und Bestrahlen mit Mikrowellen der Müll auf eine Wärmebehandlungstemperatur von über 120° C bei mindestens 2 bar (absolut) aufgeheizt und dort während einer Mindestdauer gehalten wird.

2. Verfahren nach Anspruch 1, dadurch gekennzeichnet, daß die Mindestdauer 1 bis 10 Minuten beträgt.

3. Vorrichtung zur Durchführung des Verfahrens nach Anspruch 1 oder 2 mit einer nach außen verschließbaren Baueinheit, bestehend aus einem Eingabeabschnitt mit einem Zerkleinerer und einer sich daran anschließenden Mikrowellenkammer in Form einer Durchlaufkammer mit einer Transportschnecke für eine Bewegung des Mülls entlang mehrerer, außenseitig und in Durchlaufrichtung nebeneinander angeordneter Mikrowellensender, dadurch gekennzeichnet, daß die Mikrowellenkammer (13) gebildet wird von einer Druckkammer (14) mit steuerbaren Düsen (21) sowie daran angeschlossenen Speiseleitungen (22) für ein Einlassen von Wasserdampf zur Druckeinstellung in der Mikrowellenkammer (13) und zur Temperaturregelung in Verbindung mit den Mikrowellen und mit ein- und ausgangsseitig angeordneten Absperrorganen (16, 17) zum zeitweiligen Verschließen der Mikrowellenkammer, in der die Transportschnecke (19) vor- und rückwärtsrotierend verfahrbar ist.

4. Vorrichtung nach Anspruch 3, dadurch gekennzeichnet, daß die Mikrowellenkammer (13) bei Temperaturen zwischen 130° C und 145° C betreibbar ist.

5. Vorrichtung nach Anspruch 3 oder 4, dadurch gekennzeichnet, daß die Speiseleitungen (22) für den Wasserdampf mehrere mit Abstand entlang der Mikrowellenkammer (13) verteilte Injektionsstellen (21) umfassen.

6. Vorrichtung nach einem der Ansprüche 3 bis 5, dadurch gekennzeichnet, daß die Mikrowellenkammer (13) zwischen einem Transferbehälter (9) und einer Ausförderkammer (15) für jeweils gleiche Durchsatzmengen angeordnet ist, die gemeinsam einen Sterilisations-Zyklus aus drei gleichen Chargen zum Einfüllen, Sterilisieren und Auswerfen bilden.

7. Vorrichtung nach Anspruch 6, dadurch gekennzeichnet, daß die Ausförderkammer (15) eine Entleerungsschnecke (27) aufweist.

8. Vorrichtung nach einem der Ansprüche 2 bis 7, dadurch gekennzeichnet, daß die Druckkammer (14) zur Aufnahme von Chargengrößen von 200 bis 400 l ausgelegt ist.

9. Vorrichtung nach einem der Ansprüche 2 bis 8, dadurch gekennzeichnet, daß die Druckkammer (14) ausgebildet ist als rohrförmiges Metallgehäuse mit mikrowellendurchlässigen Fenstern oder Ausschnitten, an denen ein Leitersystem der Mikrowellensender (18) angeschlossen ist.

**10.** Vorrichtung nach Anspruch 9, dadurch gekennzeichnet, daß jeweils zwei um 180° in Umfangsrichtung zueinander versetzt angeordnete Fenster oder Ausschnitte vorgesehen sind.

**FIG. 1**

**FIG. 2**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | US-A-4 861 956 (C.G. COURNEYA)<br>* Zusammenfassung *<br>--- | 1 | A 61 L 11/00 |
| D,Y | DE-C-3 505 570 (SANIMED)<br>* Ansprüche 1,2 *<br>--- | 1 | |
| A | DE-U-8 810 087 (R.E. ROLAND)<br>* Anspruch 1 *<br>----- | 1 | |

| RECHERCHIERTE SACHGEBIETE (Int. Cl.5) |
|---|
| A 61 L |

Der vorliegende Recherchenbericht wurde für alle Patentansprüche erstellt

| Recherchenort | Abschlußdatum der Recherche | Prüfer |
|---|---|---|
| DEN HAAG | 27-06-1990 | PELTRE CHR. |